# EUROPEAN PATENT APPLICATION

(11) **EP 4 841 545 A1**
(43) Date of publication of application: **18.11.2026**
(21) Application number: 25176346.2
(22) Date of filing: 14.05.2025
(51) Int. Cl.: G06Q 30/0601, G16H 20/60, C12M 1/36

(54) **SYSTEM AND METHOD FOR THE PERSONALIZATION OF FERMENTED FOOD PRODUCTS**

(71) Applicant: Millow Holding AB, 443 61 Stenkullen (SE)
(72) Inventor: Taherzadeh, Mohammad, 422 44 Hisings Backa (SE); Taherzadeh, Sajjad, 417 24 Göteborg (SE); Wainaina, Steven, 506 41 Borås (SE)

(57) **Abstract**

The invention relates to a computer-implemented method, and a corresponding system, of controlling an industrialized fermentation process to produce a personalized fermented food product. The method comprises receiving (S1) user-specific data, via an input interface, said user-specific data including at least one nutritional target comprising a recommended daily value or therapeutic threshold for a nutrient or macronutrient, and accessing (S2) a fermentation knowledge base comprising ingredient composition data specifying macronutrient and/or micronutrient profiles, measured correlations between fermentation parameters and changes in nutrient content, to thereby retrieve food production data for the fermentation process. The method further comprises executing (S3), based on the user-specific data and/or the retrieved food production data, an Artificial Intelligence, AI, based processing module that uses at least one non-linear or machine-learning model trained to predict nutrient retention in response to varying fermentation conditions, and computes fermentation settings to meet said at least one nutritional target within a target interval and/or achieve a target nutrient retention level. The method also comprises applying (S4) the fermentation settings for at least partly controlling the fermentation process.

## Description

### TECHNICAL FIELD

The present invention relates to industrialized food production, and more specifically to a computer-implemented method of controlling an industrialized fermentation process to produce a personalized fermented food product, a corresponding system and computer-readable medium as well as an industrialized food production method for producing such a personalized fermented food product, and a computer-implemented method of calibrating and validating a machine learning model used to generate personalized fermented food recipes.

### BACKGROUND

Historically, large-scale food production has been dominated by a "one-size-fits-all" approach designed to serve broad demographic segments at the lowest possible cost. Standardization has been the norm: food items are formulated to meet statistically average taste preferences and nutritional baselines, relying on cost-effective and reproducible processing methods. While effective at providing consistent, shelf-stable products, this model often ignores significant inter-individual variability in dietary needs, metabolic responses, and health conditions.

To address these limitations, recent efforts have emerged around "customization" of foods, in which consumers select among ingredient options. Examples include self-serve condiment stations, build-your-own menu concepts in cafeterias, or online ordering platforms that allow a user to add or omit certain ingredients. Although these customization approaches offer a degree of control over the final product, they typically do not address deeper biochemical transformations-especially fermentation-and rarely integrate robust health data, advanced nutrient profiling, or post-production analytics.

Over roughly the last decade, personalized nutrition-sometimes referred to as precision nutrition-has gained significant traction. Researchers in fields such as nutrigenomics, metabolomics, proteomics, and microbiome science now acknowledge that individuals can have drastically different responses to the same foods, driven by genetics, gut microbial profiles, epigenetic factors, and daily lifestyle habits. A "healthy" or "optimal" diet for one person might not produce the same benefits for another.

Accordingly, a new wave of consumer-facing services attempts to combine an individual's biometric data (like blood glucose levels, blood pressure, BMI, genotype) with general dietary guidelines to propose more precise meal plans. However, most of these services remain superficial in how they handle food manufacturing. They frequently rely on static nutrition tables, do not address complex food-processing steps such as fermentation, and offer limited feedback loops to refine recommendations based on real-world outcomes.

Thus, there is a general demand for improvements relating to personalization of food products in the technical field of industrialized food production.

### SUMMARY

It is a general object to improve certain aspects of industrialized food production.

More particularly, in industrialized fermentation processes, controlling the fermentation conditions and composition is crucial to produce high-quality products with consistent nutritional content. However, traditional methods of controlling fermentation processes often rely on manual adjustments and trial-and-error approaches, which can be time-consuming and inefficient. Furthermore, the increasing demand for personalized food products with specific nutritional profiles has created a need for more advanced control systems that can adapt to individual user requirements. Existing solutions often lack the ability to integrate user-specific data, such as nutritional targets, into the fermentation process control system. It is an object to overcome these and/or other limitations of the prior art.

Therefore, there is a need for an improved methodology that can control an industrialized fermentation process to produce personalized fermented food products, e.g., with more precise nutritional content.

It is a particular object to provide improvements related to controlling an industrialized fermentation process for producing a personalized fermented food product.

It is also an object to provide a method of calibrating and validating a machine learning model used to generate personalized fermented food recipes.

These and other objects are met by embodiments of the invention as defined in the independent claims.

According to a first aspect of the invention, there is provided a computer-implemented method of controlling an industrialized fermentation process to produce a personalized fermented food product. The method comprises:
**receiving** user-specific data, via an input interface, the user-specific data including:
   - at least one nutritional target comprising a recommended daily value or therapeutic threshold for a nutrient or macronutrient;
**accessing** a fermentation knowledge base comprising:
   - ingredient composition data specifying macronutrient and/or micronutrient profiles;
   - measured correlations between fermentation parameters and changes in nutrient content;
   to thereby retrieve, at least partly based on the user-specific data, food production data for the fermentation process;
**executing,** based on the user-specific data and/or the retrieved food production data, an Artificial Intelligence, AI, based processing module that:
   - uses at least one non-linear or machine-learning model trained to predict nutrient retention in response to varying fermentation conditions;
      and
   - computes fermentation settings to meet said at least one nutritional target within a target interval and/or achieve a target nutrient retention level; and
**applying** the fermentation settings for at least partly controlling the fermentation process.

In this way, a comprehensive, AI-driven approach to personalization of food products is enabled, considering the deeper biochemical transformations of the fermentation process itself and computing more optimal fermentation settings.

According to a second aspect of the invention, there is provided a corresponding system for Artificial Intelligence, AI, driven fermentation control to produce a personalized fermented food product.

According to a third aspect of the invention, there is provided a corresponding computer-readable medium.

According to a fourth aspect of the invention, there is provided an overall industrialized food production method for producing such a personalized fermented food product. The method comprises the computer-implemented method of controlling an industrialized fermentation process according to the first aspect.

According to a fifth aspect of the invention, there is provided a computer-implemented method of calibrating and validating a machine learning model used to generate personalized fermented food recipes. The method comprises:
**collecting** a proprietary dataset of fermentation runs, each associated with measured nutrient retention, flavor compounds, and/or cost data under systematically varied fermentation settings;
**labeling** each dataset entry with corresponding user nutritional goals and/or a measured flavor acceptability index;
**training or fine-tuning** the machine learning model to reduce error in predicting nutrient retention and/or flavor acceptability for novel ingredient compositions; and
**verifying** that the machine learning model's recommended fermentation protocol maintains a flavor acceptability index above a first target threshold and/or a nutrient retention improvement above a second target threshold.

Other advantages will be appreciated when reading the detailed description.

### BRIEF DESCRIPTION OF DRAWINGS

The embodiments, together with further objects and advantages thereof, may best be understood by making reference to the following description taken together with the accompanying drawings, in which:
FIG. 1 is a schematic diagram illustrating an example of an overall food production system as well as an integrated system for fermentation control to produce a personalized fermented food product.
FIG. 2 is a schematic flow diagram illustrating an example of a computer-implemented method of controlling an industrialized fermentation process.
FIG. 3 is a schematic flow diagram illustrating an example of additional optional steps for the computer-implemented method of FIG. 2.
FIG. 4 is a schematic flow diagram illustrating an example of a computer-implemented method of calibrating and validating a machine learning model used to generate personalized fermented food recipes.
FIG. 5 is a schematic diagram illustrating an example of a fully personalized formulation process.
FIG. 6 is a schematic diagram illustrating an example of how the system handles group formulations.
FIG. 7 is a schematic diagram illustrating an example of an overall production process, from the receipt of an order to delivery.
FIG. 8 is a diagram illustrating an example of a distribution and delivery schematic.
FIG. 9 is a schematic diagram illustrating an example of a computer implementation according to an embodiment.

### DETAILED DESCRIPTION

For a better understanding of the invention, it may be useful to start by elaborating on deeper biochemical transformations such as fermentation in food production.

Fermentation is a critical process in many global food traditions and significantly affects the nutritional and organoleptic properties of the final product:
1. Nutrient bioavailability and transformation: Fermentation can increase the bioavailability of proteins, vitamins, and minerals; reduce antinutritional factors like phytic acid; and produce bioactive metabolites (e.g., short-chain fatty acids, organic acids, antioxidants).
2. Flavor, aroma, and texture: Through the action of microbial strains (bacteria, yeasts, filamentous fungi), fermentation can yield unique flavors, aromas, and textures that are otherwise difficult to create through simpler manufacturing processes.
3. Health and functional benefits: Many fermented foods contain probiotics, prebiotics or functional compounds associated with improved gut health, immune modulation, or anti-inflammatory effects.

Despite these benefits, fermented food personalization has been underexplored in the prior art due to its complexity. Small variations in fermentation time, temperature, moisture, microbial inoculum, or substrate composition can drastically change the product's nutrient profile and flavor. Traditional "personalization" approaches-like adding or removing toppings-do not capture these dynamic transformations. Hence, personalization for fermented foods requires a data-driven system capable of predictive modeling and iterative improvements.

The inventors have recognized that the integration of cutting-edge artificial intelligence (AI) methods, including large language models (LLMs) and big-data analytics, will revolutionize the landscape of precision fermentation. By harnessing massive, heterogeneous datasets-ranging from microbial genomic sequences to real-time sensor readings-LLMs can capture nuanced relationships between fermentation parameters, microbial behaviors, and consumer feedback, thereby driving increasingly accurate predictive models and personalized product recommendations.

At the core of LLM technology lies the transformer architecture, which relies on self-attention mechanisms to identify and weigh critical features across diverse streams of input data. In the context of precision fermentation, these inputs might include textual feedback from consumers, multi-omics datasets (genomics, proteomics, metabolomics, metagenomics), or structured laboratory measurements of pH, temperature, and nutrient concentrations. Each piece of information is converted into a dense numerical embedding a high-dimensional vector representation that preserves semantic and contextual relationships. Through multiple layers of transformer blocks, the model learns how these embeddings interact, adapting to domain-specific fermentation dynamics by paying attention to the most relevant signals in each data stream.

Training such AI systems involves optimizing a loss function, often a form of cross-entropy, that measures the discrepancy between the model's predictions and ground-truth labels or numerical targets. For instance, when predicting how varying the temperature or the inoculation density might influence protein content or flavor complexity, the model refines its internal parameters through gradient descent, iteratively reducing errors across a vast array of training examples. Over successive production cycles, the system may integrate direct user feedback-such as taste preferences and nutritional requirements-back into the model, fine-tuning its embedding layers and attention weights to achieve more precise, context-aware outputs.

This closed-loop process results in adaptive recipe-generation and continuous performance improvements. For example, after each fermentation run, updated lab measurements, user-supplied sensory evaluations, and new omics data are embedded and ingested by the LLM, allowing it to recalibrate its predictions for the next cycle. Such iterative refinement enables highly targeted interventions-like adjusting fermentation times, tweaking substrate formulations, or optimizing microbial consortia-to achieve specific health-oriented or functional goals (e.g., higher probiotic viability, enhanced vitamin content, or more palatable texture). Ultimately, the synergy of LLMs, transformers, and big-data analytics provides a potent toolset for orchestrating precision fermentation, ensuring that nutritional profiles, flavor characteristics, and/or health benefits align with increasingly personalized consumer demands.

Existing patents and published applications dealing with food personalization often focus on:
Ingredient-level customization: Systems that dispense customized combinations of simple ingredients (e.g., US 8504196 B2; US 8249946 B2). While an improvement over one-size-fits-all models, these methods typically lack robust computational intelligence or focus exclusively on blending, omitting the transformative power of fermentation.

Automated dispensing or cooking: AI-based or robotic systems (e.g., US 2021/0350438 A1) that can assemble or cook ingredients automatically based on user data. Still, the emphasis is on assembly or heating rather than fermentation.

3D Food Printing: Some technologies leverage 3D printing to shape and partially customize foods. However, the emphasis typically remains on visual presentation or ingredient layering rather than the biochemical processes that yield health benefits and enhanced flavors.

Critically, the prior art does not disclose a holistic system that combines user data, advanced AI modeling, and/or real-time feedback loops specifically tailored to state-state fermentation or related fermentation strategies.

The present invention bridges that gap by uniting domain-specific databases (fermentation parameters, ingredient compositions), big-data analytics, and self-learning algorithms to deliver truly personalized fermented foods.

From an industrial and commercial standpoint, the development and production of personalized fermented foods represent a pivotal advancement in the functional food sector. By leveraging cutting-edge bioprocessing technologies, these products can be tailored to meet specific dietary profiles and physiological needs. For instance, athletes may benefit from high-protein, low-carbohydrate formulations optimized for muscle recovery and metabolic efficiency, while individuals diagnosed with conditions such as celiac disease or lactose intolerance require hypoallergenic or lactose-free varieties that still deliver essential probiotic and nutritional benefits. In geriatric populations, easily digestible, nutrient-dense formulations help compensate for age-related declines in gastrointestinal motility and nutrient absorption, while also accommodating diminished olfactory acuity (hyposmia) and gustatory perception (hypogeusia) to ensure adequate intake of essential nutrients.

An important aspect relates to the integration of automated, data-driven manufacturing pipelines. By way of example, machine learning algorithms and real-time sensing technologies enable precise control over fermentation parameters-such as pH, temperature, microbial consortia, and nutrient composition-thereby ensuring consistency and scalability. This data-centric approach allows manufacturers to rapidly prototype new formulations, streamline production workflows, and maintain rigorous quality assurance standards. As a result, the personalized fermentation sector can serve diverse niche markets at scale, establishing a new class of value-added, health-focused products.

On a broader societal level, the diffusion of personalized fermented foods has potential to meaningfully influence population-wide health outcomes. Diet-related non-communicable diseases, including obesity, type 2 diabetes, and cardiovascular disorders, are often linked to suboptimal nutritional patterns and micronutrient deficiencies. By harnessing real-time data-collected from wearables, electronic health records, and user feedback-these adaptive fermentation processes can be continuously refined to accommodate different metabolic requirements and dietary restrictions. This iterative model of food production aligns with emerging paradigms in personalized nutrition and precision medicine, which emphasize the tailoring of dietary interventions to individual genetic, microbiome, and lifestyle parameters.

The capacity to provide semi-personalized or group-personalized fermented meals at scale is particularly relevant for institutional settings such as schools, hospitals, and nursing homes. Through integrated supply chain systems and robust data analytics, these institutions can procure and distribute targeted, nutrient-optimized fermented foods to larger populations. This approach not only improves diet quality and addresses specific health risks within these communities but also generates valuable data for public health surveillance. By correlating consumption patterns, health metrics, and microbial profiles, stakeholders can refine both clinical interventions and policy decisions in near real-time.

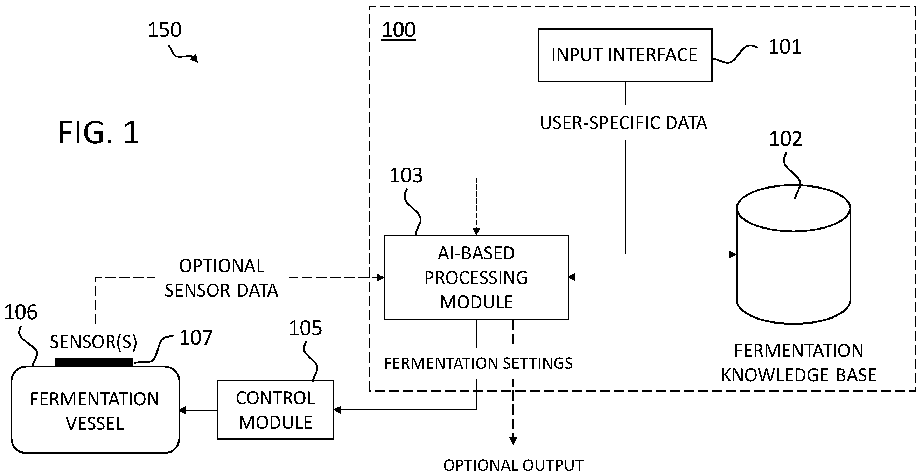

FIG. 1 is a schematic diagram illustrating an example of an overall food production system 150 as well as an integrated AI-driven system 100 for fermentation control to produce a personalized fermented food product.

Basically, the system 100 for controlling an industrialized fermentation process comprises an input interface 101, a fermentation knowledge base 102 and an AI-based processing module 103.

The system 100 may also be referred to as system for AI-driven fermentation control, and it may interact with a control module 105 that controls the fermentation process in a fermentation vessel 106.

The system 100 for controlling an industrialized fermentation process may also interact with optional sensor data from one or more sensors 107 associated with the fermentation vessel 106.

The AI-based processing module 103, which may be referred to as an AI-based decision engine may compute the fermentation settings and optionally also output a fermentation protocol and/or a food production recipe.

According to a first aspect of the invention, there is provided a computer-implemented method of controlling an industrialized fermentation process to produce a personalized fermented food product.

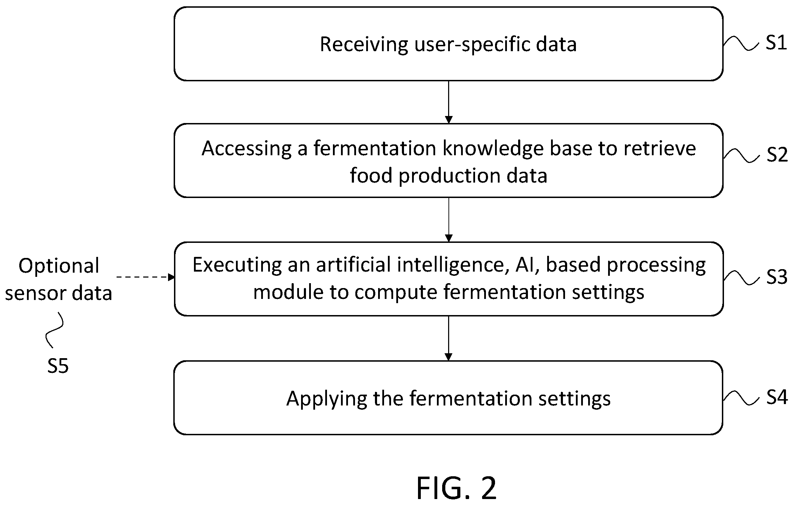

With reference to FIG. 2, the method comprises:
step S1: **receiving** user-specific data, via an input interface, the user-specific data including:
   - at least one nutritional target comprising a recommended daily value or therapeutic threshold for a nutrient or macronutrient;
step S2: **accessing** a fermentation knowledge base comprising:
   - ingredient composition data specifying macronutrient and/or micronutrient profiles;
   - measured correlations between fermentation parameters and changes in nutrient content;
   to thereby retrieve, at least partly based on the user-specific data, food production data for the fermentation process;
step S3: **executing,** based on the user-specific data and/or the retrieved food production data, an Artificial Intelligence, AI, based processing module that:
   - uses at least one non-linear or machine-learning model trained to predict nutrient retention in response to varying fermentation conditions;
      and
   - computes fermentation settings to meet said at least one nutritional target within a target interval and/or achieve a target nutrient retention level; and
step S4: **applying** the fermentation settings for at least partly controlling the fermentation process.

In this way, a comprehensive, AI-driven approach to personalization of food products is enabled, considering the deeper biochemical transformations of the fermentation process itself and computing more optimal fermentation settings.

Preferably, the AI-based processing module is executed based on the retrieved food production data and optionally at least part of the user-specific data.

By way of example, the fermentation settings or parameters relate to temperature, pH, duration and/or humidity.

For example, it may be desirable to achieve a nutrient retention level that is at least 10% greater than a baseline static fermentation process.

In a particular example, the user-specific data further includes at least one cost constraint, and the fermentation knowledge base comprises process cost data correlating ingredient usage and fermentation conditions, and the retrieved food production data includes data derived based on the process cost data. In this example, the AI-based processing module integrates the at least one user-specific nutritional target and the at least one cost constraint, and computes fermentation settings to meet the at least one nutritional target within a target interval and/or achieve a target nutrient retention level, while meeting the at least one cost constraint.

Optionally, the user-specific data may further comprise additional user-specific data including at least one of the following:
- demographic information;
- health or medical condition data;
- dietary preferences and known allergies.

In this example, the food production data for the fermentation process retrieved from the fermentation knowledge base may also be derived at least partly based on the additional user-specific data.

As an example, the computer-implemented method of controlling an industrialized fermentation process may further comprise:
collecting (S5) real-time sensor data from at least one fermentation vessel; and
executing (S3) the AI-based processing module also based on at least part of said real-time sensor data to enable real-time tracking of macronutrient changes and/or flavor compound formation to more accurately maintain the at least one nutritional target and/or the target nutrient retention level; and/or
automatically adjusting, via a control interface, at least one fermentation parameter of the fermentation settings in real time in response to deviations detected in the sensor data, thereby dynamically preserving the at least one nutritional target and/or the target nutrient retention level.

For example, the real-time sensor data may be taken at intervals of less than 15 minutes. The real-time sensor data may include, e.g., at least one of temperature, pH, humidity, and microbial load or growth rate.

By way of example, the fermentation vessel may be equipped with multi-spectral or near-infrared sensors for estimating nutrient composition in situ, and the sensor readings may be fed into the AI-based processing module, enabling non-invasive real-time tracking of macronutrient changes and/or flavor compound formation to more accurately maintain a user's nutrient retention threshold(s).

In a specific example, which may be particularly advantageous, the fermentation knowledge base comprises flavor compound references that map measured compound levels to a predetermined flavor acceptability index. In this example, the AI-based processing module uses at least one non-linear or machine-learning model trained to predict nutrient retention and flavor development in response to varying fermentation conditions, and computes the fermentation settings to meet the at least one nutritional target within a target interval and/or achieve a target nutrient retention level, while meeting said at least one cost constraint and maintaining a flavor acceptability index above a target value.

In a particular, non-limiting example, the flavor acceptability index scaled from 0 to 100, and a target is to maintain a flavor acceptability index ≥75.

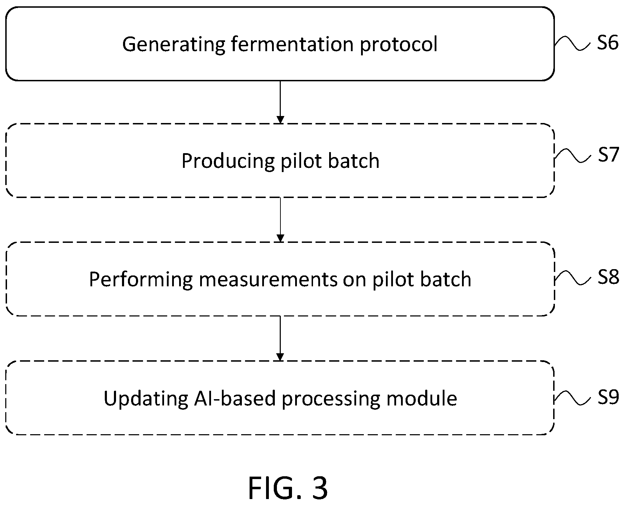

With reference to the non-limiting example of FIG. 3, the computer-implemented method may further comprise the step S6 of generating an optimized fermentation protocol specifying:
- substrate composition, including ingredient proportions;
- at least one of microorganism strain selection and inoculation density;
- at least one real-time fermentation parameter schedule; and/or
- any post-fermentation step required for stability or nutrient fortification.

Optionally, the computer-implemented method further comprises:
step S7 of producing a pilot batch in a laboratory or small-scale production environment using the optimized fermentation protocol;
step S8 of measuring actual nutrient composition, a flavor acceptability index, and/or at least one textural attribute of the pilot batch; and
step S9 of updating the AI-based processing module by incorporating the measured data, wherein the module retrains or fine-tunes a predictive model to reduce a discrepancy between predicted and actual fermentation outcomes in subsequent production runs.

By way of example, the method described herein may be applied to:
- an individual user to produce a personalized fermented food product customized for that individual user; or
- a group of users sharing at least one common dietary or health constraint to produce a personalized fermented food product customized for that group of users.

It should thus be understood that the term "personalized" encompasses both individual and group applications. It is indeed a widely used term in the context of healthcare and also consumer products. The idea is to provide a customizable procedure for providing a fermentation process and corresponding personalized fermented food product that can be tailored or customized to the specific needs of each user, irrespective of whether they are an individual or part of a group. Sometimes, the term "user-centric" may be used interchangeably with the term "personalized" to cover both individual use or group applications.

In a particular example, the AI-based processing module clusters or segments users into subgroups having at least one common dietary or health constraint, and autogenerates a respective fermentation protocol variant for each subgroup, each variant satisfying at least one of:
- a flavor acceptability index above a target level,
- a nutrient retention level improved by at least a predetermined percentage relative to a static process, and
- an ingredient or operational cost within each subgroup's defined cost threshold.

By way of example, the AI-based processing module may select from a plurality of microorganism strains including at least one of bacterial cultures and filamentous fungi a blend of at least two strains, the blend being chosen to synergistically enhance nutrient bioavailability by at least an additional 5% beyond a single-strain protocol, as verified by comparing final nutrient-retention outcomes in a test run.

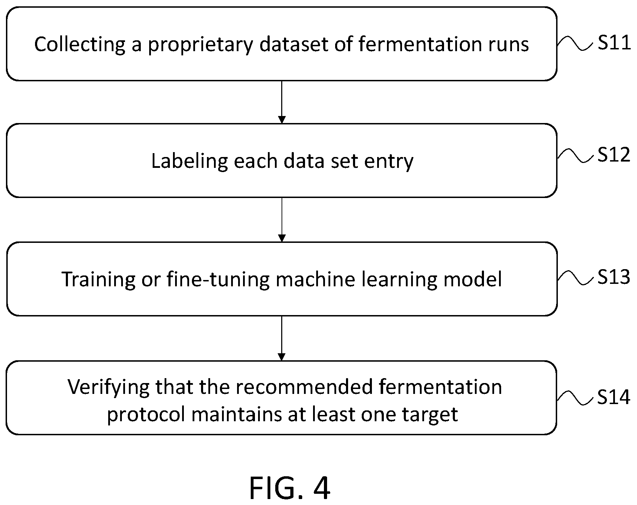

With reference to FIG. 4, there is illustrated a computer-implemented method of calibrating and validating a machine learning model used to generate personalized fermented food recipes.

The method comprises:
step S11 of **collecting** a proprietary dataset of fermentation runs, each associated with measured nutrient retention, flavor compounds, and/or cost data under systematically varied fermentation settings;
step S12 of **labeling** each dataset entry with corresponding user nutritional goals and/or a measured flavor acceptability index;
step S13 of **training or fine-tuning** the machine learning model to reduce error in predicting nutrient retention and/or flavor acceptability for novel ingredient compositions;
step S14 of **verifying** that the machine learning model's recommended fermentation protocol maintains a flavor acceptability index above a first target threshold and/or a nutrient retention improvement above a second target threshold.

For example, each fermentation run may automatically be tagged with traceability metadata indicating timestamps, sensor readings, user constraints, and final product metrics, enabling regulatory compliance verification and progressive improvement of the model's predictions across multiple production batches.

The proposed technology also provides an overall food production method for producing a personalized fermented food product, wherein the industrialized food production method comprises the computer-implemented method of controlling an industrialized fermentation process described herein.

With reference once again to FIG. 1, the invention provides a system 100 for Artificial Intelligence, AI, driven fermentation control to produce a personalized fermented food product, wherein the system is configured to perform the method of controlling an industrialized fermentation process described herein.

By way of example, the system 100 and/or the overall food production system 150 comprises:
- an input interface 101 configured to receive the user-specific data;
- a fermentation knowledge base 102 configured to store ingredient composition data, microbial strain profiles, nutritional guidelines, and previously measured fermentation outcomes;
- a sensor suite 107, embedded in at least one fermentation vessel 106, configured to measure sensor data related to the fermentation process;
- an AI-based processing module 103 implementing the at least one non-linear or machine-learning model, responsive to the sensor data, to generate, as output, real-time adjustments of fermentation parameters; and
- a control module 105 configured to physically modulate fermentation conditions in accordance with the output of the AI-based processing module.

Optionally, the system further comprises a feedback module (not shown in FIG. 1) that is configured to:
collect post-production data on actual nutrient levels, flavor acceptability scores, and/or cost parameters; and
continuously update the AI-based processing module by adjusting at least one machine-learning weight or process parameter correlation, thereby iteratively improving the accuracy of recipe generation in subsequent fermentations.

In a fully comprehensive embodiment, the proposed technology offers a method of dynamically controlling an industrialized fermentation process in real-time to produce a personalized fermented food product meeting user-specified nutritional, organoleptic and cost constraints.

By way of example, the AI-based processing module may be configured as a multi-objective optimization module, preferably for executing a machine-learning model.

In general, AI-based procedures involving machine learning can be based on artificial neural networks or similar architectures. Learning can be supervised, semi-supervised or unsupervised.

In a particular example, the data processing may be configured to perform the data processing by means of a trained neural network, support vector machine or decision-tree based implementation.

In general, neural networks, also commonly referred to as artificial neural networks, can be regarded as computing systems vaguely inspired by the biological neural networks that constitute the brain. The neural network itself is not an algorithm, but rather a framework for many different machine learning algorithms to work together and process complex data inputs. In a way, such systems "learn" to perform tasks by considering examples, generally without being programmed with any task-specific rules. A neural network may be based on a collection of connected units or nodes called artificial neurons, which loosely model the neurons in a biological brain. Each connection, like the synapses in a biological brain, can transmit a signal from one artificial neuron to another. An artificial neuron that receives a signal can process it and then signal additional artificial neurons connected to it. In common implementations, the signal at a connection between two artificial neurons can be represented by a real number, and the output of each artificial neuron may be computed by some non-linear function of the sum of its inputs. The connections between artificial neurons are called 'edges'. Artificial neurons and edges typically have a weight that adjusts as learning proceeds. The weight increases or decreases the strength of the signal at a connection. Artificial neurons may have a threshold such that the signal is only sent if the aggregate signal crosses that threshold. Typically, artificial neurons are aggregated into layers. Different layers may perform different kinds of transformations on their inputs. Signals travel from the first layer (the input layer), to the last layer (the output layer), possibly after traversing the layers multiple times. Many different types of more evolved neural networks have been developed over time, for example convolutional neural networks, recurrent neural networks and hierarchical neural networks.

The adjective "deep" in deep learning originates from the use of multiple layers in the network. Early work showed that a linear perceptron cannot be a universal classifier, and that a network with a non-polynomial activation function with one hidden layer of unbounded width can on the other hand so be. Deep learning is a modern variation which is concerned with an unlimited number of layers of bounded size, which permits practical application and optimized implementation, while retaining theoretical universality under mild conditions. In deep learning the layers are also permitted to be heterogeneous and to deviate widely from biologically informed connectionist models, for the sake of efficiency, trainability and understandability.

By way of example, the AI-based processing module may be trained according to the following guidelines:
In a first, data-curation stage, a domain-specific corpus is assembled that (i) ingests peer-reviewed articles, patents and regulatory monographs on food chemistry and fermentation, (ii) streams high-frequency sensor signals-temperature, pH, dissolved-oxygen, gas-chromatography traces-captured from pilot-scale fermenters, and (iii) stores time-stamped still images and hyperspectral frames of microbial growth phases. The textual records are converted to dense embeddings and, together with the sensor and image embeddings, are persisted in a managed, cloud-native vector database that supports approximate-nearest-neighbor search across tens of billions of vectors with sub-second latency. The same cloud platform also hosts state-of-the-art transformer-based foundation models (e.g., language models of 70 B parameters or more and complementary vision encoders) and exposes them through a uniform REST/HTTPS inference and fine-tuning API.

During training, a dual-tower encoder is constructed in which (a) the language foundation model is parameter-efficiently fine-tuned via low-rank adaptation (LoRA rank ≤ 64) on the curated textual corpus, while (b) a vision transformer ingests the image and time-series sensor snapshots. The two towers are co-trained with a contrastive loss so that heterogeneous modalities occupy a shared metric space, thereby enabling cross-modal retrieval. A compound objective is optimized that combines (i) next-token likelihood on domain language, (ii) mean-squared error between predicted and measured nutrient-retention vectors, and (iii) a contrastive alignment term that minimizes the distance between retrieved passages and aligned sensor-image embeddings.

At inference time, a retrieval-augmented-generation (RAG) loop first queries the vector database to obtain the top-k semantically nearest passages and multimodal embeddings relevant to the user-specified nutritional targets and current fermenter telemetry. These retrieved artifacts are injected into the context window of the fine-tuned language model, which then produces candidate fermentation settings. An integrated "agent" framework within the cloud platform orchestrates this loop end-to-end: it autonomously invokes internal REST and OPC-UA production APIs, evaluates hard safety and allergen constraints, pulls real-time tank telemetry, and iterates the prompt chain until convergence on the personalized nutritional objectives.

Model performance is continuously monitored through on-line shadow deployments in which live production recommendations are replayed against a hold-out validation set; any distributional drift exceeding a predefined Kullback-Leibler divergence threshold automatically triggers a retraining cycle that re-executes the foregoing pipeline and promotes the updated model to production after passing regression and canary tests.

For a better understanding, the proposed technology will now be described and discussed with reference to additional non-limiting exemplifying embodiments.

The present invention provides a comprehensive, AI-driven system and method for controlling and/or producing personalized fermented food products.

By way of example, the proposed technology may integrate databases, one or more AI-based processing modules, production workflow and/or distribution and delivery.

### 1. Databases:

For example, this system comprises one or more interlinked databases that collectively support personalized product development. First, the ingredients database characterizes the nutritional composition, flavor profiles, color, texture, and cost of cereals, legumes, spices, vegetables, herbs, and nutritional supplements. Second, the fermentation effects database captures how critical parameters-such as temperature, duration, humidity, and inoculum type-impact the final product's composition, flavor, texture, antinutrients and concentration of bioactive compounds. Third, the health effects database provides guidance on dietary recommendations, possible restrictions (such as allergies or intolerances), and potential interactions with medications or lifestyle factors. Finally, an available recipes database contains validated or "default" formulations, ranging from broad categories (e.g., athletic performance or geriatric nutrition) to specialized recipes created in previous personalization tasks.

### 2. AI-Based decision engine:

The AI-based Decision Engine can be implemented as a multi-layer system that integrates advanced machine learning methods, knowledge-based repositories, and a secure, user-friendly interface. First, a data ingestion layer collects and normalizes inputs from various sources-such as medical prescriptions, health metrics, lifestyle tracking devices, and user preferences-to ensure consistency across all data fields. This information is then stored in a centralized repository (for example, a relational database or a knowledge graph) that maps relevant dietary constraints, flavor and texture preferences, and recipe data, enabling efficient retrieval and comparison.

On top of this repository, predictive models-ranging from traditional statistical approaches to deep learning architectures-identify near-matching recipes from the database or generate new formulations when none exist. These models are specifically tailored to account for key parameters such as nutritional requirements, fermentation conditions, and desired flavor profiles. To ensure continuous improvement, the system includes a feedback loop that evaluates production outcomes, laboratory test results, and consumer satisfaction metrics, allowing the AI to refine its predictions over time.

A dynamic optimization layer further fine-tunes the Engine's recommendations by weighing multiple objectives-cost, taste, nutritional balance, and health constraints-so that each new recipe or formulation meets highly individualized goals. Throughout this process, privacy and security considerations remain paramount, with sensitive user data anonymized or pseudonymized in accordance with regulatory guidelines. As more user feedback is integrated, the models become increasingly accurate and personalized, ultimately delivering safer, more effective, and more enjoyable dietary solutions.

### 3. Production workflow:

When a novel or extensively modified formulation is proposed, the process initiates with a pilot-scale production phase carried out under strictly controlled conditions. During this stage, comprehensive organoleptic assessments-including evaluations of flavor profiles, olfactory characteristics, and rheological properties-are conducted in conjunction with advanced laboratory analyses. These analyses measure nutrient composition and verify the presence or concentration of target metabolites, thereby validating and refining the AI's predictive algorithms. Upon successful completion of these assessments and any necessary adjustments, the formulation proceeds to the scale-up phase, where it is authorized for routine large-scale production.

### 4. Distribution and delivery:

Distribution and delivery processes can be strategically optimized by allowing orders to be placed either by individual consumers or by designated group representatives, such as those overseeing nutritional programs in educational institutions, directing dietary plans in healthcare facilities, or managing meal solutions for professional sports teams. By segmenting these groups according to shared nutritional profiles, dietary restrictions, and organoleptic preferences-encompassing factors like taste, texture, and aroma-multiple semi-personalized formulations can be developed to meet the specific palatal and physiological requirements of each subgroup. Depending on logistical constraints, final products may then be provided in various formats, including fresh, frozen, or pre-cooked offerings. This flexible approach not only accommodates diverse storage capacities and transportation considerations but also preserves product quality and bioavailability of nutrients, ensuring that every meal adheres to rigorous food safety standards and aligns with the precise demands of modern nutritional science.

Through this invention, consumers receive tailored fermented foods that more precisely match their health status, personal tastes, and budget constraints, while producers benefit from a robust system that leverages data-driven insights to innovate rapidly, minimize waste, and continuously optimize for quality and consistency.

The following description explains how exemplifying embodiments of the invention constructs, manages, and utilizes multiple data repositories in conjunction with AI-based modeling to create personalized fermented food products. Various embodiments are described, including solid-state fermentation for high-protein plant substrates, multi-strain probiotic fermentations for functional foods, and group personalization for institutional clients.

### I. Data Infrastructure

### A. Ingredients Database

### 1. Composition and nutritional data

Each ingredient (cereals, legumes, vegetables, herbs, spices, nutritional additives) has a detailed profile capturing macro- and micronutrient content (e.g., proteins, carbohydrates, lipids, vitamins, minerals, fiber). In certain embodiments, the database also links to textual descriptors (e.g., "nutty flavor," "earthy aroma") derived from sensory panel evaluations or publicly available organoleptic data.

### 2. Price and availability

Real-time or periodically updated data on ingredient cost, seasonal availability, and supply chain metrics are also recorded. This enables cost-optimized recipe generation for budget-conscious consumers.

### 3. Allergen information

Each ingredient record includes allergen data (e.g., "contains soy," "gluten-free," "nut-free facility"), ensuring that AI-generated recipes immediately screen out or adapt potential allergens based on user restrictions.

### B. Fermentation Effects Database

### 1. Parameter-Outcome Mappings

The Fermentation Effects Database serves as a comprehensive repository for understanding how varying operational parameters-such as temperature, relative humidity, aeration rates, inoculum density, fermentation duration, and substrate composition-influence the outcomes of solid-state fermentation. By methodically mapping each parameter to its specific impact, this database reveals the intricate biochemical and enzymatic pathways responsible for altering the nutritional profile (including amino acid composition and vitamin retention), generating or modulating flavor compounds (such as aldehydes, esters, and organic acids), shaping textural attributes (such as firmness, moisture content, and cohesiveness), and inducing color variations (through processes like enzymatic browning or pigment formation). These insights enable researchers and practitioners to fine-tune fermentation conditions, thereby optimizing both the nutritional integrity and sensorial appeal of the final product while advancing the scientific understanding of microbial metabolism and its practical applications.

### 2. Strain-Level Details

The Strain-Level Details component of the Fermentation Effects Database systematically captures the unique attributes of microbial cultures frequently utilized in fermentative processes, including edible filamentous fungi (e.g. from genera *Rhizopus, Aspergillus*), yeasts (e.g., *Saccharomyces cerevisiae*) or lactic acid bacteria. This module compiles in-depth growth kinetics for each strain, enabling precise calibration of fermentation parameters to optimize yield and quality. It also documents a broad spectrum of metabolites produced by these microorganisms-such as short-chain fatty acids, amino acids, and polyphenols-thereby elucidating critical biochemical pathways that govern nutritional and functional outcomes. Furthermore, the database identifies and catalogs the distinctive flavor and aroma compounds associated with each strain's "sensory fingerprint," offering valuable insights for achieving targeted product profiles and advancing the science of microbial-driven food innovation.

### 3. Dynamic Learning

Dynamic Learning is a continuously adaptive feedback mechanism that enhances the precision and robustness of the fermentation effects database over time. As practical fermentation runs are conducted, comprehensive laboratory analyses are carried out to quantify critical parameters, including nutrient composition, pH trajectories, microbial population dynamics, and the production of metabolic byproducts. These empirically derived datasets are then systematically incorporated into the database, thereby updating the parameter-outcome correlations and refining advanced AI-driven predictive models. Through this iterative cycle of data ingestion and computational modeling, the platform not only improves its predictive accuracy for future fermentations but also uncovers new insights into microbial consortia interactions and the emergent properties of complex fermentation systems. Ultimately, this dynamic approach enables both researchers and industry practitioners to optimize process parameters, ensuring consistent product quality and paving the way for innovative applications of fermentation science.

### C. Health Effects Database

### 1. Recommended daily values

The health effects database incorporates region-specific recommended dietary allowances (RDAs), dietary reference intakes (DRIs), and broader guidelines issued by organizations such as the World Health Organization (WHO) and the Food and Agriculture Organization (FAO). These recommended values serve as evidence-based benchmarks for optimal nutrient intake and are derived from comprehensive analyses of population health data, metabolic studies, and international consensus among nutrition scientists.

To enhance accuracy and applicability, these daily intake recommendations are further stratified according to demographic parameters such as age, sex, and health status. Special consideration is given to individuals who have increased physiological requirements, including pregnant or nursing women, as well as older adults who may exhibit altered nutrient metabolism. By adapting the guidelines in this manner, the database can provide precise dietary targets that align with current scientific understanding of nutritional needs across diverse populations. This nuanced approach allows healthcare professionals, policy makers, and researchers to make informed decisions regarding dietary recommendations, fortification programs, and personalized nutrition interventions.

### 2. Functional compounds and dietary restrictions

The database provides a comprehensive overview of functional compounds and the associated dietary restrictions necessary for specific health objectives. Functional additives-ranging from microbial strains such as probiotics designed to enhance gut microbiome diversity, to nutraceutical ingredients like curcumin and resveratrol-are highlighted for their documented bioactive properties and potential to support various physiological functions, including immune modulation, inflammatory response regulation, and antioxidant activity. By cataloging these functional substances alongside the latest clinical and epidemiological research, the database enables healthcare professionals, nutritionists, and food scientists to formulate evidence-based products tailored to specific health needs and consumer demographics.

In parallel, the database addresses critical dietary restrictions by outlining hypoallergenic protocols, diabetic-friendly guidelines (including formulations with reduced glycemic indices), and low-sodium recommendations for individuals at risk of or managing hypertension. Through this targeted approach, users can identify ingredients that meet both functional and safety criteria, ensuring that dietary products optimize health while conforming to clinical best practices. By integrating this dual focus on functional compounds and specialized nutritional requirements, the database promotes the development of scientifically grounded, user-centric dietary solutions.

### 3. Drug-food interactions

A vital component of the invention is its capacity to detect and account for clinically significant interactions between food constituents and various pharmacological agents. For example, high levels of vitamin K, commonly found in fermented legumes, can potentially antagonize the anticoagulant effects of certain blood-thinning medications. Utilizing a robust pharmacological knowledge base, the AI within this system can automatically flag these potential conflicts, prompting either a reformulation of the product or an adjustment to the recommended consumption guidelines. In doing so, the invention fosters a safer and more personalized dietary framework-particularly crucial for individuals on complex medication regimens or those managing chronic conditions-by mitigating adverse events and supporting evidence-based nutritional planning.

### D. Available Recipes Database

### 1. Default or baseline formulations

The system incorporates a robust repository of scientifically validated formulations designed to address the diverse nutritional needs of broad demographic groups. These "default" or baseline recipes undergo thorough biochemical and organoleptic assessments to ensure optimal macronutrient balance, micronutrient density, and overall palatability. For example, one default formulation is a high-protein, fermented legume-based blend specifically tailored to athletes or highly active individuals who require elevated protein intake for muscle repair and development. This recipe leverages controlled fermentation techniques to enhance protein bioavailability, reduce anti-nutritional factors (such as phytic acid), and improve gut microbiome health. Another baseline formulation is a gentle, easily digestible fermented cereal aimed at older adults who may benefit from milder textures and reduced gastrointestinal load. By employing carefully selected cereal grains and probiotics, this formulation supports nutrient absorption and addresses age-related changes in digestive function. All baseline recipes are subject to ongoing evaluation and refinement, integrating current research on human nutrition, fermentation science, and population-specific dietary requirements to maintain safety, efficacy, and consumer acceptance.

### 2. Archived user-specific recipes

Whenever a user or consumer group completes a personalized recipe, the system securely archives a comprehensive dataset detailing the formulation. This dataset includes selected ingredients, precise fermentation parameters, laboratory test results (for nutrient composition and safety), cost analysis, and aggregated consumer feedback. By systematically recording these data points, the platform enables evidence-based evaluations and rapid retrieval through advanced indexing and machine-learning techniques. As a result, future consumers with similar nutritional profiles, health goals, or dietary constraints can be matched to previously validated formulations before the system invests resources in generating entirely new recipes. This approach not only streamlines the personalization process but also enhances overall consistency, cost-efficiency, and user satisfaction.

### 3. Multi-criteria tagging

Each recipe is systematically annotated with a robust set of metadata tags, such as "gluten-free," "vegan," "low-GI" (low glycemic index), or "high-fermentable-fiber," to streamline the discovery and adaptation process for new users. This multi-criterion tagging framework is grounded in established nutritional guidelines and food science principles, enabling swift filtering and customization based on specific dietary restrictions, health goals, or personal preferences. By employing a standardized taxonomy for these tags, the system ensures consistent recipe classification and accelerates both user-driven searches and automated recommendation algorithms.

### II. Al-Driven Personalization Engine

### A. Input gathering

The AI-driven personalization engine employs a robust, multifaceted data collection process to generate precise, individualized nutritional recommendations. Data are systematically gathered through secure interfaces-such as web portals, mobile applications, or specialized kiosks-and encompass a broad spectrum of parameters. Demographic details, including age, sex, weight, and body mass index (BMI), serve as foundational health indicators, while clinical data, such as blood glucose levels, lipid profiles, known diseases (e.g., diabetes, celiac disease), and current medications, refine the engine's capacity to account for metabolic constraints and specific dietary requirements. Lifestyle factors, ranging from occupational exertion levels to structured physical activity and weekly sports schedules, enable the system to align caloric intake with actual energy expenditure. Concurrently, personal preferences are captured through organoleptic variables-taste, color, and texture-and the system automatically flags any allergens or strongly disliked ingredients to optimize user satisfaction and adherence.

Furthermore, special conditions such as pregnancy, hospitalization, or the dietary needs of nursing-home residents trigger custom constraints, exemplified by recommendations to exclude raw-sprout fermentations for immunocompromised individuals. By integrating these diverse data inputs, the AI-driven personalization engine applies advanced machine learning algorithms to deliver scientifically grounded, dynamic, and context-sensitive dietary strategies tailored to each user's evolving health status, lifestyle demands, and personal preferences.

### B. Recipe proposal algorithm

### 1. Matching to existing recipes

In this initial phase, the AI-driven system compares the consumer's profile-encompassing dietary preferences, allergen restrictions, and specific nutritional goals-to a comprehensive library of previously validated formulations. By applying advanced pattern-matching and machine learning techniques, the algorithm calculates a "similarity score" based on parameters such as macronutrient ratios, micronutrient targets, and known allergen constraints. If the system identifies a similarity score at or above a predetermined threshold (commonly 85%), it concludes that an existing recipe sufficiently fulfills the consumer's requirements. Recommending a recipe already present in the database not only streamlines the product development cycle but also utilizes a formulation with a proven track record of success, thus minimizing both risk and resource expenditure.

### 2. Generating a new recipe

When the similarity score does not meet the threshold, the AI proceeds to develop a novel recipe tailored to the consumer's specific needs. This process begins by filtering ingredients in the database to eliminate allergens and optimize macronutrient and micronutrient levels. Concurrently, it consults the fermentation effects database to identify suitable fermentation protocols-i.e., microbial strains, fermentation time, and temperature-that can induce the desired biochemical transformations, thereby enhancing nutrient bioavailability or altering flavor profiles. The system also cross-references the health effects database to ensure all critical medical considerations and dietary constraints are integrated. Finally, the AI virtually "tests" the proposed formulation, iteratively refining factors such as ingredient concentrations, fermentation duration, and supplementary additives until simulated nutritional and sensory outcomes align with the user's stated goals and preferences.

### 3. Cost and feasibility assessment

Once a recipe proposal-be it an existing formulation or a newly generated concept-has been established, the AI conducts a thorough cost and feasibility analysis. Leveraging real-time data on ingredient pricing, supply chain capacity, and production facility constraints, the system evaluates the economic viability of the formulation. If cost projections exceed acceptable thresholds or if specific ingredients and microbial strains are unavailable, the AI autonomously proposes substitution options that maintain comparable nutritional and functional attributes. This approach ensures not only that the resulting product meets consumer health and sensory requirements but also that it remains feasible within budgetary and logistical limitations.

### 4. Presentation to user

The final step involves presenting the "optimal recipe" to the consumer, and optionally, to a medical professional for additional validation. Through an intuitive user interface, the system provides a comprehensive overview of projected nutritional values-including protein, carbohydrate, and fat content, as well as associated vitamins, minerals, and estimated glycemic index. In addition, organoleptic descriptors such as flavor notes, expected texture, and aroma are highlighted to offer insights into the sensorial experience. Accompanying this information is a detailed breakdown of cost estimates and a proposed production timeline, enabling the consumer to make an informed decision that balances health objectives, taste preferences, and budgetary considerations.

### C. Adaptive and self-learning mechanisms

### 1. Post-production feedback

Once a product has been manufactured and consumed, the AI-driven platform gathers a range of post-production data to enhance its future recipe development. This process typically involves collecting consumer feedback through surveys or direct input channels, focusing on metrics such as taste satisfaction, texture perception, and perceived health benefits. Simultaneously, any remaining product samples are subjected to laboratory analyses that measure changes in nutrient composition, microbial viability, pH level, and other relevant physiochemical parameters. By integrating both subjective user responses and objective scientific data, the system refines its predictive models and machine learning algorithms. Over time, this feedback loop continually improves recipe accuracy, ensuring that subsequent formulations are better aligned with consumer preferences and nutritional objectives.

### 2. Continuous integration of new scientific data

In order to maintain the cutting edge of innovation, the system actively monitors and incorporates emerging findings in fermentation science, nutrition, and related fields. Research discoveries-ranging from novel microbial strains with unique metabolic pathways to groundbreaking enzymatic processes-are systematically reviewed by R&D specialists. Upon validation, these insights are then fed into the platform's databases and algorithmic frameworks. This ongoing integration ensures that the AI remains current with the latest scientific advancements, thereby expanding its ability to recommend new functional ingredients, adapt to specialized dietary requirements, and refine production processes. As a result, the system evolves in real time, guided by a robust scientific foundation that continually pushes the boundaries of nutritional product development.

### III. Production workflow

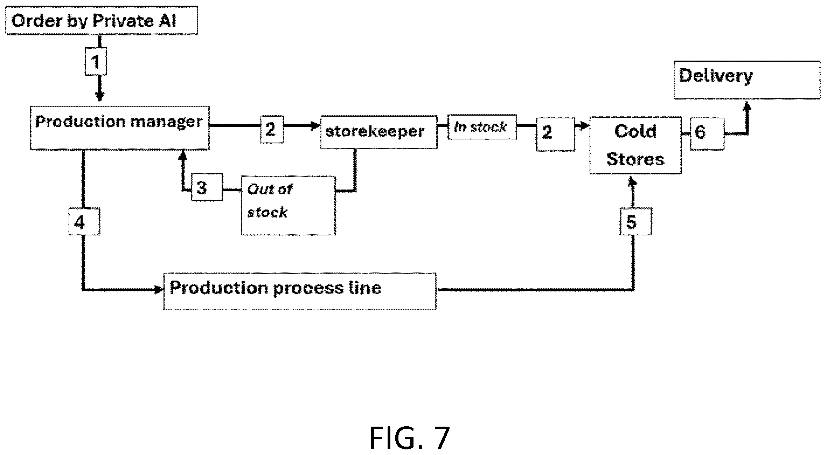

With reference to FIG. 7, there is illustrated a schematic example of a production process, from the receipt of an order to delivery. By way of example, FIG. 7 details an example of the mixing and weighing of ingredients, sterilization, shaping, fermentation, and final packaging steps, highlighting the importance of in-stock vs. out-of-stock ingredients and the storekeeper's role in supply chain management.

Once an order is placed-whether for an individual consumer or a larger batch request-it is transmitted to the production manager's queue, as illustrated in FIG. 7. The system evaluates the recipe specifications to determine if a pilot run is necessary. In particular, heavily adapted or newly developed recipes may require a preliminary test to verify processing parameters, optimize ingredient ratios, and ensure that the production line can reliably replicate the desired characteristics of the final product. Data from these pilot runs can then be integrated into predictive models and stored for future reference.

Ingredients are confirmed for availability, precisely weighed, and goes through the entire production line including pretreatment, solid-state fermentation and post-treatment. Final product assessment involves a thorough yet streamlined Quality Assurance (QA) protocol. If the product is made using a new recipe or a substantially modified one, the QA team may conduct expanded testing-monitoring parameters like microbial load, sensory attributes, nutritional composition, and physical integrity. Once the batch meets predefined acceptance criteria, it is packaged in materials that maintain product integrity and comply with regulatory requirements.

In brief, FIG. 7 highlights the storekeeper's role in maintaining ingredient stock, the production manager's oversight, and the points of quality control. It concludes with the packaging step and final readiness for delivery.

### IV. Group personalization

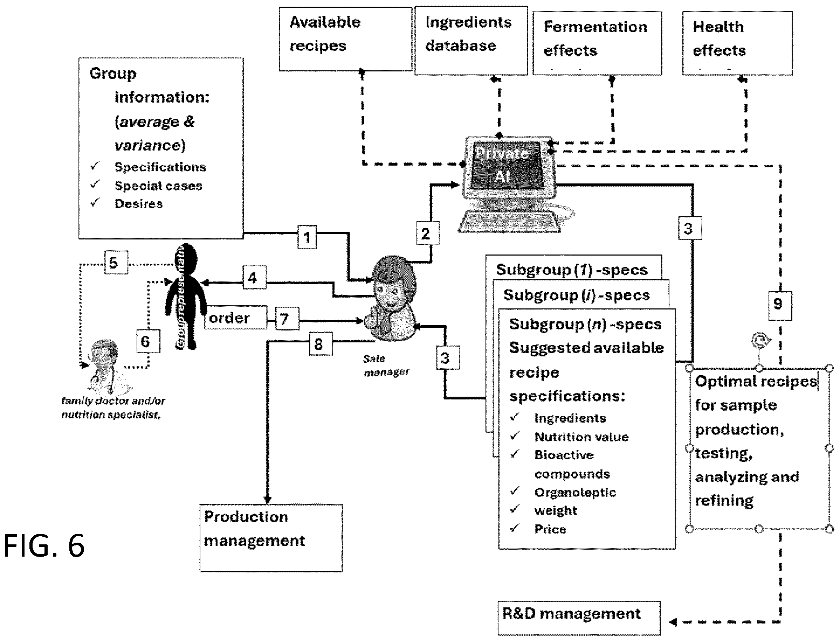

With reference to FIG. 6, there is illustrated a flowchart depicting an example of how the system may handle group formulations. It outlines an example of how group information - averages, variances, special dietary restrictions - is processed by the AI. The result is a set of subgroup-specific recipes, each with distinct nutrient and flavor profiles. The figure also illustrates the role of the sale manager, production management, and R&D management in orchestrating the personalization for multiple consumers at once.

For larger institutions-such as schools, long-term care facilities, or hospitals-the system applies sophisticated group-level analytics to streamline recipe design and production, as depicted in FIG. 6. By aggregating and analyzing anthropometric data (e.g., average weight, age distribution), as well as relevant nutritional biomarkers (e.g., protein status, lipid profiles, micronutrient levels), the AI engine segments individuals into subgroups based on shared nutritional priorities. Examples of these targeted subgroups include those requiring low-sodium formulations, high-protein regimens, or allergen-free meal components.

Once these subgroups are delineated, the system generates specialized "semi-personalized" recipes for each category, striking a balance between individualized nutritional adequacy and the operational feasibility of large-scale production. Instead of attempting to produce fully customized meals for each member of a large population-which would be logistically complex and cost-prohibitive-the algorithm deploys an optimized suite of recipes tailored to the distinct metabolic, clinical, and sensory needs identified for each subgroup. This method leverages real-time updates from ingredient databases and leverages health effects data to ensure that each recipe aligns with professional dietary guidelines and safety standards, while simultaneously accommodating group-specific palatability and convenience requirements.

In brief, FIG. 6 shows how the system may process group-level data such as average age, weight, special cases like celiac disease or hypertension. The AI may then partition a large group into subgroups (e.g., Subgroup 1, Subgroup i, Subgroup n), each of which receives a suggested recipe.

### V. Research & Development

### A. Baseline ("default") recipe development

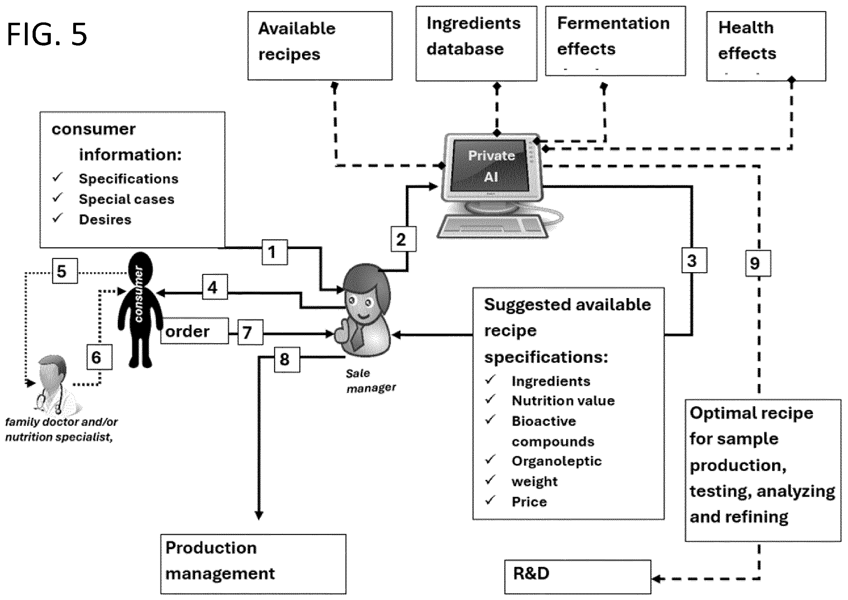

With reference to FIG. 5, there is shown a flowchart illustrating an example of a fully personalized formulation process.

FIG. 5 specifically illustrates how customer-provided data - such as age, health conditions, and flavor or texture preferences - flows into a private AI system. The AI references multiple product specifications (including ingredients, fermentation parameters, health impacts, nutritional profiles, sensory attributes, and cost) to identify the product most likely to satisfy the customer's criteria, effectively minimizing predictive error (i.e., achieving a near-zero loss function). The recommended product is then forwarded to the production line. In parallel, the AI evaluates any discrepancies between the customer's desired attributes and the available products, prompting R&D to investigate and refine factors that could further optimize future product offerings.

By way of example, consumer data (specifications, special cases, flavor/color/texture desires) is fed into the AI, which references various databases (ingredients, fermentation effects, health effects, available recipes) to propose candidate formulations. FIG. 5 also depicts potential interactions with the family doctor or nutrition specialist, as well as the role of R&D managers and production managers in refining and validating the "optimal recipe."

Research & Development (R&D) teams may establish a foundational library of "default" recipes aimed at common demographic categories, such as weight-management formulas, high-energy formulations, and gluten-free alternatives. These baseline recipes undergo iterative testing to validate their nutritional composition, organoleptic properties (flavor, texture, aroma, appearance), and microbial stability under standard production conditions. Once validated, they form a robust starting point for more specialized or personalized requests. By continually refining this default recipe bank, the system ensures that new product iterations can leverage established nutritional and culinary best practices, expediting the overall customization process for diverse user requirements.

### B. Laboratory analysis

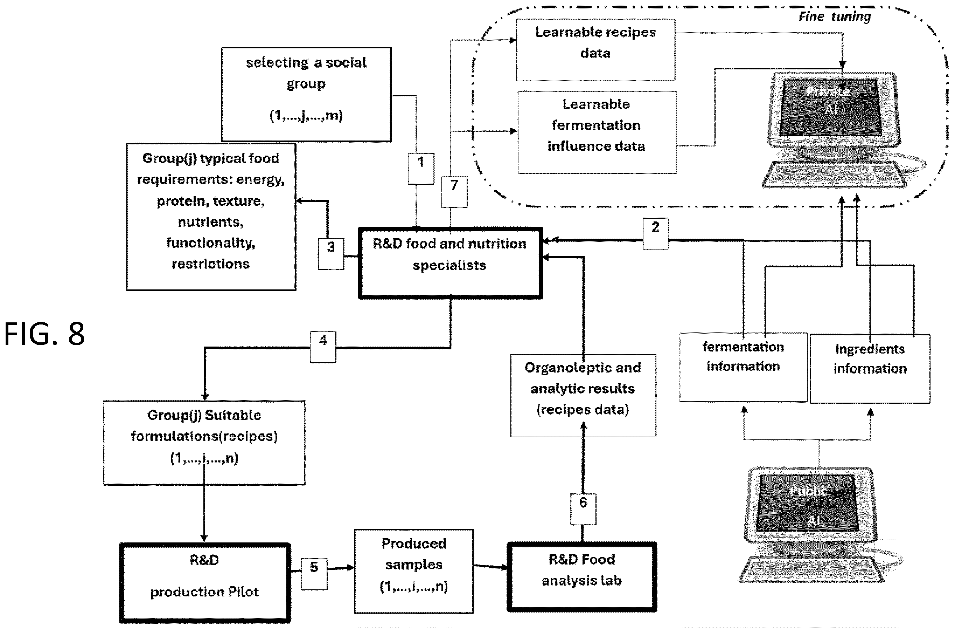

Following the R&D phase, a dedicated food analysis laboratory (refer to both FIG. 5 and the iterative sampling steps in FIG. 8) conducts comprehensive evaluations of each pilot or final product. This analytical process quantifies macronutrients (proteins, carbohydrates, and fats) as well as micronutrients (vitamins and minerals), thus confirming the recipe's alignment with targeted dietary guidelines. In parallel, the lab measures functional or bioactive compounds (e.g., probiotics, antioxidants) that can confer health benefits beyond basic nutrition. Organoleptic qualities-ranging from taste descriptors to mouthfeel and texture-are assessed using standardized sensory panels and instrumental texture analysis. Additionally, microbiological safety is verified through pathogen screening to maintain strict adherence to food safety regulations. These data points are systematically integrated into the fermentation and health effects databases, enabling the AI-driven model to continuously refine its predictions regarding final product attributes and viability.

### C. Continuous fine-tuning

With reference to FIG. 8, there is illustrated an example of a distribution and delivery schematic, overlaying a high-level "recipe development flowchart" for selecting a social group, analyzing typical requirements, generating pilot products, and refining them based on organoleptic and analytic results from the lab. It emphasizes the cyclical "learnable fermentation influence data" that the AI uses to refine its predictive models.

In a sense, FIG. 8 is a higher-level diagram that combines the concept of selecting a social group and carrying out R&D production pilots with the iterative "learnable fermentation influence data". After pilot batches are produced for a social group, lab analyses and feedback are uploaded into the Private AI. Over time, the system "fine tunes" its ability to create new, specialized recipes for further groups or individuals.

The system applies a cyclical development model where pilot batches for emerging demographic groups or subgroups are produced and subsequently tested in the laboratory. The analytical results-including nutritional composition, bioactive compound levels, and fermentation performance-feed back into the AI's central databases, allowing for near-real-time recalibration of predictive algorithms. This iterative process ensures that the model's parameters remain in sync with empirical findings, thereby enhancing the precision of future recipe formulations. Over time, this loop reduces production costs and material waste while also decreasing the time-to-market for new product variations, ultimately driving the invention's capability to deliver consistent, high-quality, and scientifically validated food solutions.

### VI. Example use case

An example scenario illustrates how the invention operates end to end:
1. User profile: A 55-year-old female user with borderline high blood pressure (hypertension), a preference for mildly savory flavors, and an allergy to soy. She also wants a moderate protein intake for muscle maintenance.
2. AI recommendation: The system filters out soy-based substrates and checks the fermentation database for suitable legumes or cereals with high protein but moderate sodium. It proposes a blend of lentils (for protein) and barley (for beneficial beta-glucans). A *Rhizopus* strain is selected for fermentation to enhance digestibility and produce a subtle savory profile.
3. Recipe presentation: The user sees an on-screen readout:
   - Estimated nutritional values (protein ~15 g per serving, moderate sodium, essential vitamins).
   - Organoleptic notes ("lightly chewy texture, mild umami flavor").
   - Approximate price and production timeline.
4. Production: After user confirmation, the batch is prepared in the facility, right recipe is selected, upstream process, fermentation and downstream process are carried out according to the predefined factors that are dictated by research and development (R&D) unit.
5. Delivery and feedback: The fermented product is delivered. The user provides feedback on taste, texture, and satisfaction. The system logs any adjustments needed (e.g., "slightly too chewy, or too salty") and updates the user's profile, refining the AI for future orders. These data are delivered to R&D for possible development of a new recipe according to the desired properties.

It will be appreciated that the methods and procedures and arrangements described herein can be implemented, combined and re-arranged in a variety of ways.

For example, embodiments may be implemented in hardware, or at least partly in software for execution by suitable processing circuitry, or a combination thereof.

The steps, functions, procedures, and/or blocks described herein may be implemented in hardware using any conventional technology, such as discrete circuit or integrated circuit technology, including both general-purpose electronic circuitry and application-specific circuitry.

Alternatively, or as a complement, at least some of the steps, functions, procedures, and/or blocks described herein may be implemented in software such as a computer program for execution by suitable processing circuitry such as one or more processors or processing units.

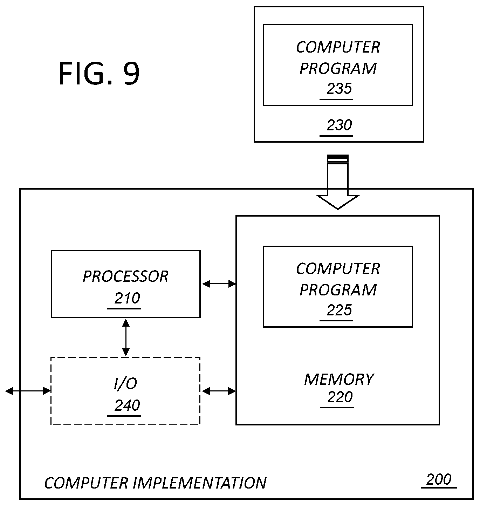

FIG. 9 is a schematic diagram illustrating an example of a computer implementation according to an embodiment. In this particular example, the system 200 comprises a processor 210 and a memory 220, the memory comprising instructions executable by the processor, whereby the processor is operative to perform the steps and/or actions described herein. The instructions are typically organized as a computer program 225; 235, which may be preconfigured in the memory 220 or downloaded from an external memory device 230. Optionally, the system 200 comprises an input/output interface 240 that may be interconnected to the processor(s) 210 and/or the memory 220 to enable input and/or output of relevant data such as input parameter(s) and/or resulting output parameter(s).

The term 'processor' should be interpreted in a general sense as any system or device capable of executing program code or computer program instructions to perform a particular processing, determining or computing task.

The processing circuitry including one or more processors is thus configured to perform, when executing the computer program, well-defined processing tasks such as those described herein.

The processing circuitry does not have to be dedicated to only execute the above-described steps, functions, procedure and/or blocks, but may also execute other tasks.

The proposed technology also provides a computer-program product comprising a computer-readable medium 220; 230 having stored thereon such a computer program.

According to an aspect of the invention, there is thus provided a non-transitory computer-readable medium storing instructions that, when executed by one or more processors, cause the processors to perform the method described herein.

By way of example, the instructions may comprise:
code for receiving and storing the user-specific input data;
code for accessing the fermentation knowledge base containing ingredient, sensor, and nutritional guidelines;
code for executing a trained AI-based multi-objective model that predicts nutrient composition changes, flavor acceptability, and/or cost implications under varying fermentation conditions;
code for performing real-time adjustments to fermentation parameters; and
code for outputting a final optimized fermentation protocol meeting user-specified nutrition, flavor, and/or cost goals.

By way of example, the software or computer program 225; 235 may be realized as a computer program product, which is normally carried or stored on a computer-readable medium 220; 230, in particular a non-volatile medium. The computer-readable medium may include one or more removable or non-removable memory devices including, but not limited to a Read-Only Memory (ROM), a Random Access Memory (RAM), a Compact Disc (CD), a Digital Versatile Disc (DVD), a Blu-ray disc, a Universal Serial Bus (USB) memory, a Hard Disk Drive (HDD) storage device, a flash memory, a magnetic tape, or any other conventional memory device. The computer program may thus be loaded into the operating memory of a computer or equivalent processing device for execution by the processing circuitry thereof.

Method flows may be regarded as a computer action flows, when performed by one or more processors. A corresponding device, system and/or apparatus may be defined as a group of function modules, where each step performed by the processor corresponds to a function module. In this case, the function modules are implemented as a computer program running on the processor. Hence, the device, system and/or apparatus may alternatively be defined as a group of function modules, where the function modules are implemented as a computer program running on at least one processor.

The computer program residing in memory may thus be organized as appropriate function modules configured to perform, when executed by the processor, at least part of the steps and/or tasks described herein.

Alternatively, it is possible to realize the modules predominantly by hardware modules, or alternatively by hardware. The extent of software versus hardware is purely implementation selection.

The embodiments described above are merely given as examples, and it should be understood that the proposed technology is not limited thereto. It will be understood by those skilled in the art that various modifications, combinations and changes may be made to the embodiments without departing from the present scope as defined by the appended claims. In particular, different part solutions in the different embodiments can be combined in other configurations, where technically possible.

## Claims

1. A computer-implemented method of controlling an industrialized fermentation process to produce a personalized fermented food product, the method comprising:
**receiving** (S1) user-specific data, via an input interface, said user-specific data including:
- at least one nutritional target comprising a recommended daily value or therapeutic threshold for a nutrient or macronutrient;
**accessing** (S2) a fermentation knowledge base comprising:
- ingredient composition data specifying macronutrient and/or micronutrient profiles;
- measured correlations between fermentation parameters and changes in nutrient content;
to thereby retrieve, at least partly based on the user-specific data, food production data for the fermentation process;
**executing** (S3), based on the user-specific data and/or the retrieved food production data, an Artificial Intelligence, AI, based processing module that:
- uses at least one non-linear or machine-learning model trained to predict nutrient retention in response to varying fermentation conditions;
and
- computes fermentation settings to meet said at least one nutritional target within a target interval and/or achieve a target nutrient retention level; and
**applying** (S4) the fermentation settings for at least partly controlling the fermentation process.

2. The computer-implemented method of controlling an industrialized fermentation process according to claim 1, wherein said user-specific data further includes at least one cost constraint,
wherein the fermentation knowledge base comprises process cost data correlating ingredient usage and fermentation conditions, and the retrieved food production data includes data derived based on the process cost data; and
wherein the AI-based processing module integrates said at least one user-specific nutritional target and said at least one cost constraint, and computes fermentation settings to meet said at least one nutritional target within a target interval and/or achieve a target nutrient retention level, while meeting said at least one cost constraint.

3. The computer-implemented method of controlling an industrialized fermentation process according to claim 1 or 2, wherein said user-specific data further comprises additional user-specific data including at least one of the following:
- demographic information;
- health or medical condition data;
- dietary preferences and known allergies; and
wherein the food production data for the fermentation process retrieved from the fermentation knowledge base is also derived at least partly based on said additional user-specific data.

4. The computer-implemented method of controlling an industrialized fermentation process according to any of the claims 1 to 3, wherein said method further comprises:
collecting (S5) real-time sensor data from at least one fermentation vessel; and
executing (S3) the AI-based processing module also based on at least part of said real-time sensor data to enable real-time tracking of macronutrient changes and/or flavor compound formation to more accurately maintain said at least one nutritional target and/or said target nutrient retention level; and/or automatically adjusting, via a control interface, at least one fermentation parameter of the fermentation settings in real time in response to deviations detected in the sensor data, thereby dynamically preserving said at least one nutritional target and/or said target nutrient retention level.

5. The computer-implemented method of controlling an industrialized fermentation process according to claim 4, wherein the fermentation vessel is equipped with multi-spectral or near-infrared sensors for estimating nutrient composition in situ, and
wherein the sensor readings are fed into the AI-based processing module, enabling non-invasive real-time tracking of macronutrient changes and/or flavor compound formation to more accurately maintain a user's nutrient retention threshold(s).

6. The computer-implemented method of controlling an industrialized fermentation process according to any of the claims 1 to 5, wherein said fermentation knowledge base comprises flavor compound references that map measured compound levels to a predetermined flavor acceptability index, and
wherein said AI-based processing module uses at least one non-linear or machine-learning model trained to predict nutrient retention and flavor development in response to varying fermentation conditions, and computes the fermentation settings to meet said at least one nutritional target within a target interval and/or achieve a target nutrient retention level, while meeting said at least one cost constraint and maintaining a flavor acceptability index above a target value.

7. The computer-implemented method of controlling an industrialized fermentation process according to any of the claims 1 to 6, further comprising:
generating (S6) an optimized fermentation protocol specifying:
- substrate composition, including ingredient proportions;
- at least one of microorganism strain selection and inoculation density;
- at least one real-time fermentation parameter schedule; and/or
- any post-fermentation step required for stability or nutrient fortification.

8. The computer-implemented method of controlling an industrialized fermentation process according to claim 7, further comprising:
producing (S7) a pilot batch in a laboratory or small-scale production environment using the optimized fermentation protocol;
measuring (S8) actual nutrient composition, a flavor acceptability index, and/or at least one textural attribute of the pilot batch; and
updating (S9) the AI-based processing module by incorporating the measured data,
wherein the module retrains or fine-tunes a predictive model to reduce a discrepancy between predicted and actual fermentation outcomes in subsequent production runs.

9. The computer-implemented method of controlling an industrialized fermentation process according to any of the claims 1 to 8, wherein the method is applied to:
- an individual user to produce a personalized fermented food product customized for that individual user; or
- a group of users sharing at least one common dietary or health constraint to produce a personalized fermented food product customized for that group of users.

10. The computer-implemented method of controlling an industrialized fermentation process according to any of the claims 1 to 9, wherein the AI-based processing module clusters or segments users into subgroups having at least one common dietary or health constraint, and autogenerates a respective fermentation protocol variant for each subgroup, each variant satisfying at least one of:
- a flavor acceptability index above a target level,
- a nutrient retention level improved by at least a predetermined percentage relative to a static process, and
- an ingredient or operational cost within each subgroup's defined cost threshold.

11. The computer-implemented method of controlling an industrialized fermentation process according to any of the claims 1 to 10, wherein the AI-based processing module selects from a plurality of microorganism strains including at least one of bacterial cultures and filamentous fungi a blend of at least two strains, the blend being chosen to synergistically enhance nutrient bioavailability by at least an additional5% beyond a single-strain protocol, as verified by comparing final nutrient-retention outcomes in a test run.

12. A system (100) for Artificial Intelligence, AI, driven fermentation control to produce a personalized fermented food product, wherein the system is configured to perform the method of controlling an industrialized fermentation process according to any of the claims 1 to 11.

13. The system (100) according to claim 12, wherein the system (100) comprises:
- an input interface (101) configured to receive the user-specific data;
- a fermentation knowledge base (102) configured to store ingredient composition data, microbial strain profiles, nutritional guidelines, and previously measured fermentation outcomes;
- a sensor suite (107), embedded in at least one fermentation vessel (106), configured to measure sensor data related to the fermentation process;
- an AI-based processing module (103) implementing the at least one non-linear or machine-learning model, responsive to the sensor data, to generate, as output, real-time adjustments of fermentation parameters;
- a control module (105) configured to physically modulate fermentation conditions in accordance with the output of the AI-based processing module.

14. The system according to claim 13, further comprising a feedback module configured to:
collect post-production data on actual nutrient levels, flavor acceptability scores, and/or cost parameters; and
continuously update the AI-based processing module by adjusting at least one machine-learning weight or process parameter correlation, thereby iteratively improving the accuracy of recipe generation in subsequent fermentations.

15. A non-transitory computer-readable medium (220; 230) storing instructions that, when executed by one or more processors (210), cause the processors (210) to perform the method according to any of the claims 1 to 11.

16. The non-transitory computer-readable medium (220; 230) according to claim 15, wherein the instructions comprises:
code for receiving and storing the user-specific input data;
code for accessing the fermentation knowledge base containing ingredient, sensor, and nutritional guidelines;
code for executing a trained AI-based multi-objective model that predicts nutrient composition changes, flavor acceptability, and/or cost implications under varying fermentation conditions;
code for performing real-time adjustments to fermentation parameters; and
code for outputting a final optimized fermentation protocol meeting user-specified nutrition, flavor, and/or cost goals.

17. A computer-implemented method of calibrating and validating a machine learning model used to generate personalized fermented food recipes, the method comprising:
**collecting** (S11) a proprietary dataset of fermentation runs, each associated with measured nutrient retention, flavor compounds, and/or cost data under systematically varied fermentation settings;
**labeling** (S12) each dataset entry with corresponding user nutritional goals and/or a measured flavor acceptability index;
**training or fine-tuning** (S13) the machine learning model to reduce error in predicting nutrient retention and/or flavor acceptability for novel ingredient compositions; and
**verifying** (S14) that the machine learning model's recommended fermentation protocol maintains a flavor acceptability index above a first target threshold and/or a nutrient retention improvement above a second target threshold.

18. The method according to claim 17, wherein each fermentation run is automatically tagged with traceability metadata indicating timestamps, sensor readings, user constraints, and final product metrics, enabling regulatory compliance verification and progressive improvement of the model's predictions across multiple production batches.

19. An industrialized food production method for producing a personalized fermented food product, wherein said industrialized food production method comprises the computer-implemented method of controlling an industrialized fermentation process according to any of the claims 1 to 11.
